(19) **Europäisches Patentamt / European Patent Office / Office européen des brevets**

(11) **EP 2 796 159 A1**

(12) **EUROPEAN PATENT APPLICATION**

(43) Date of publication:
**29.10.2014 Bulletin 2014/44**

(21) Application number: **14165978.9**

(22) Date of filing: **25.04.2014**

(51) Int Cl.:
***A61M 5/315*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.04.2013 JP 2013094655**

(71) Applicant: **Fujifilm Corporation**
**Minato-ku**
**Tokyo 106-8620 (JP)**

(72) Inventors:
• **Kawasaki, Hidetoshi**
  **Kanagawa (JP)**
• **Hatakeyama, Koichi**
  **Kanagawa (JP)**

(74) Representative: **HOFFMANN EITLE**
**Patent- und Rechtsanwälte**
**Arabellastrasse 4**
**81925 München (DE)**

(54) **Gasket device for pre-filled syringe**

(57) A gasket device is for use in a pre-filled syringe having a cylinder (11) for containing liquid drug (LM) in sealing tightness. The liquid drug is pressurized by moving a plunger (14) in the cylinder. The gasket device includes a gasket body (21, 51), having resiliency, and movable toward the liquid drug with a front end (14a) of the plunger. A laminate layer (22, 52) is formed from PTFE (polytetrafluoroethylene), and disposed to cover a surface of the gasket body. The laminate layer includes a sealing portion (22a, 52a), having a thickness Da, for sliding on an inner surface (18) of the cylinder in sealing tightness. A liquid receiving portion (22b) has a thickness Db, for contacting the liquid drug in the cylinder. The thickness Da is from 10 microns to 40 microns. The thickness Db is from 20 microns to 50 microns. A ratio R1 of the thickness Db to the thickness Da is from 1.25 to 2.00.

F I G . 2

**Description**

BACKGROUND OF THE INVENTION

1. Field of the Invention

The present invention relates to a gasket device for a pre-filled syringe. More particularly, the present invention relates to a gasket device which operates for sealing in a pre-filled syringe, and with which sealing tightness and property of inhibiting dissolution can be maintained in combination.

2. Description Related to the Prior Art

A pre-filled syringe is pre-loaded with liquid drug. The pre-filled syringe includes a cylinder, a tip cap (top cap), and a gasket device. The cylinder is a barrel used also for transport and preservation of the liquid drug. The tip cap tightly closes a needle holder, which is formed at a distal end of the cylinder. The gasket device is contained in the cylinder, is in sealing contact with an inner surface of the cylinder air-tightly and liquid-tightly in the cylinder. In injection, an injection needle is mounted in the needle holder. A plunger is mounted on the gasket device, and is driven to slide the gasket device in the cylinder. According to the use of the pre-filled syringe, it is unnecessary to load a syringe with the liquid drug from a container, so that the handlability of the syringe is high and contamination of the liquid drug can be prevented.

JP-A 2010-142573 discloses an example of the gasket device including a gasket body (sealing material) and a laminate layer (coating layer) of PTFE film (polytetrafluoroethylene film). The gasket body is formed from rubber with resiliency. The PTFE film has a low frictional coefficient and is chemically inert. Forming the laminate layer prevents dissolution of plasticizer and other additives in the gasket body into the liquid drug, and facilitates slide by keeping the frictional coefficient low for the gasket device in relation to the inner surface of the cylinder.

JP-A 2004-248985 discloses a syringe in which seal packing can be slid smoothly in the cylinder even upon pushing the plunger with an inclination. In a manner similar to the gasket device disclosed in the above document, the seal packing of JP-A 2004-248985 has the laminate layer of the PTFE film formed on the surface of the gasket body of rubber with resiliency. The seal packing contacts the inner surface of the cylinder in a linear contact in a circumferential direction, to lower frictional resistance of the gasket device with the inner surface of the cylinder. According to the document, a composite sheet material is prepared, inclusive of a resilient rubber layer and a fluorocarbon layer for the laminate layer, and is pressed by molds to obtain the seal packing by press forming. The laminate layer includes a liquid receiving portion or stopper portion (plug portion), and a slidable sealing portion or sealing membrane. The liquid receiving portion receives the liquid drug. The sealing portion contacts the inner surface of the cylinder. In the course of the press forming, the sealing portion is stretched more than the liquid receiving portion, and is formed at a smaller thickness than the liquid receiving portion.

In the gasket device for the pre-filled syringe, the sealing portion should have a small thickness for keeping sufficient sealing tightness, including air-tightness and liquid-tightness. The liquid receiving portion should have a large thickness for keeping a property of inhibiting dissolution of components in the gasket body into the liquid drug, such as a rubber component, cross-linking agents, filler, pigment, antioxidant, trace metals, non-soluble fine particles and the like. The thicknesses of the sealing portion and the liquid receiving portion should be predetermined with adjustment. In a manner similar to the seal packing of JP-A 2004-248985, the press forming is a widely used method for forming the gasket device. The laminate layer is formed by stretching the PTFE film of a single film or layer in the press forming for a thickness required for the purpose. However, should the PTFE film be overstretched in the press forming, a crack or pinhole may be created in the laminate layer, to decrease the sealing tightness and property of inhibiting dissolution. There is no known gasket device in which the liquid receiving portion and the sealing portion in the laminate layer are formed with adjusted thicknesses or in which the sealing tightness and property of inhibiting dissolution are maintained in combination.

SUMMARY OF THE INVENTION

In view of the foregoing problems, an object of the present invention is to provide a gasket device which operates for sealing in a pre-filled syringe, and with which sealing tightness and property of inhibiting dissolution can be maintained in combination.

In order to achieve the above and other objects and advantages of this invention, a gasket device for a pre-filled syringe including a cylinder for containing liquid drug in sealing tightness is provided, the liquid drug being pressurized by moving a plunger in the cylinder. The gasket device includes a gasket body, having resiliency, and movable toward the liquid drug with a front end of the plunger. A laminate layer is formed from fluorocarbon resin, and disposed to cover a surface of the gasket body. The laminate layer includes a sealing portion, having a first thickness, for sliding on an inner surface of the cylinder in sealing tightness. A liquid receiving portion has a second thickness, for contacting the liquid drug in the cylinder. The first thickness is equal to or more than 10 microns and equal to or less than 40 microns, the second thickness is equal to or more than 20 microns and equal to or less than 50 microns, and a ratio of the second thickness to the first thickness is equal to or more than 1.25 and equal to or less than 2.00.

Preferably, the laminate layer includes a transition portion formed between the sealing portion and the liquid receiving portion. The transition portion is so formed as to satisfy a condition:

$$\Delta D/L \le 1/4$$

where L is a local distance between two points arranged in the transition portion close to one another in a direction of transition, and $\Delta D$ is a thickness change in the transition portion between the two points.

Preferably, the laminate layer is in a film form, and shaped in compliance with a profile line of the gasket body by press forming of the fluorocarbon resin.

Preferably, the first thickness is smaller than the second thickness, and a thickness difference between parts of the sealing portion symmetrically opposite to each other with respect to a center line of the gasket body is equal to or less than 5 microns.

Preferably, the gasket body is formed from rubber.

Preferably, the gasket body includes first and second ring portions arranged in a moving direction of the plunger. A small diameter portion is disposed between the first and second ring portions in a layer arrangement, and has a smaller diameter than the first and second ring portions. The sealing portion is formed on a peripheral surface of each one of the first and second ring portions.

Preferably, the laminate layer is formed by press forming, and thereafter the gasket body is formed together with the laminate layer.

In another preferred embodiment, the laminate layer is formed simultaneously with forming of the gasket body.

In still another preferred embodiment, the gasket body is formed by insert molding on the laminate layer.

Consequently, sealing tightness and property of inhibiting dissolution can be maintained in combination, because the thicknesses of the sealing portion and the liquid receiving portion are conditioned suitably for cooperation.

BRIEF DESCRIPTION OF THE DRAWINGS

The above objects and advantages of the present invention will become more apparent from the following detailed description when read in connection with the accompanying drawings, in which:

Fig. 1 is a vertical section illustrating a pre-filled syringe having a gasket device;
Fig. 2 is a vertical section illustrating the gasket device;
Fig. 2A is an explanatory view illustrating a transition portion in a laminate layer;
Figs. 3A-3E are vertical sections illustrating a process of producing the gasket device;
Fig. 4 is a vertical section illustrating press forming of a rubber sheet and PTFE film;
Figs. 5A and 5B are vertical sections illustrating another preferred process of producing the gasket device by insert molding;
Fig. 6 is a vertical section illustrating another preferred gasket device having two slidable sealing portions.

DETAILED DESCRIPTION OF THE PREFERRED EMBODIMENT(S) OF THE PRESENT INVENTION

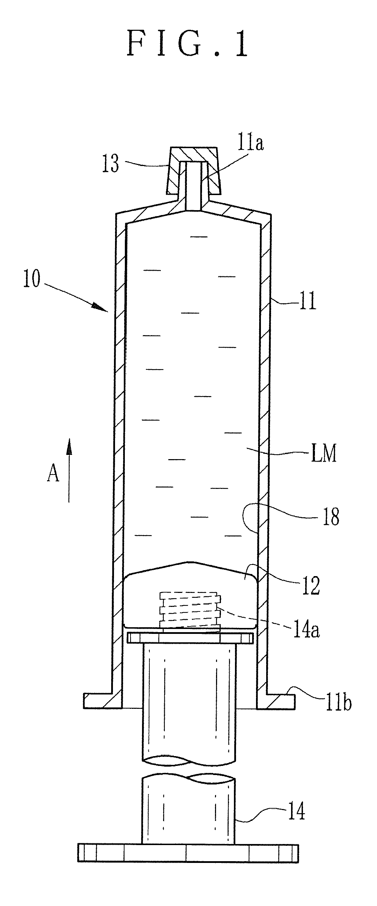

In Fig. 1, a pre-filled syringe 10 of the invention is illustrated, and includes a cylinder 11 or barrel, a gasket device 12 or plunger stopper (plunger head), a tip cap 13 (top cap), and a plunger 14. Liquid drug LM is previously loaded in the cylinder 11.

The cylinder 11 is a barrel including a needle holder 11a and a syringe flange 11b. The needle holder 11a is disposed at a distal end with a smaller diameter, and used for holding an injection needle. The cylinder 11 is formed from glass or resin, for example, polypropylene, cyclic polyolefin and the like. The tip cap 13 is fitted on the needle holder 11a and closes an end opening of the cylinder 11 air-tightly and liquid-tightly. The tip cap 13 is removable. To use the pre-filled syringe 10, the tip cap 13 is removed before the injection needle (not shown) is fitted in the needle holder 11a.

The gasket device 12 has an outer diameter originally larger than an inner diameter of the cylinder 11, and is inserted in the cylinder 11 by deformation in a radial direction with resiliency. The gasket device 12 seals the inside of the cylinder 11 air-tightly and liquid-tightly while the cylinder 11 is charged with a liquid drug LM. The gasket device 12 is slidable axially in a moving direction A with the arrow in the cylinder 11, and pressurizes the liquid drug LM to inject this through the injection needle upon sliding toward a distal end. The gasket device 12 operates as a seal packing.

Before use, the plunger 14 is preserved in a state removed from the gasket device 12. The plunger 14 is coupled to the gasket device 12 for use. The plunger 14 is driven to slide forwards to move the gasket device 12 in the moving direction A.

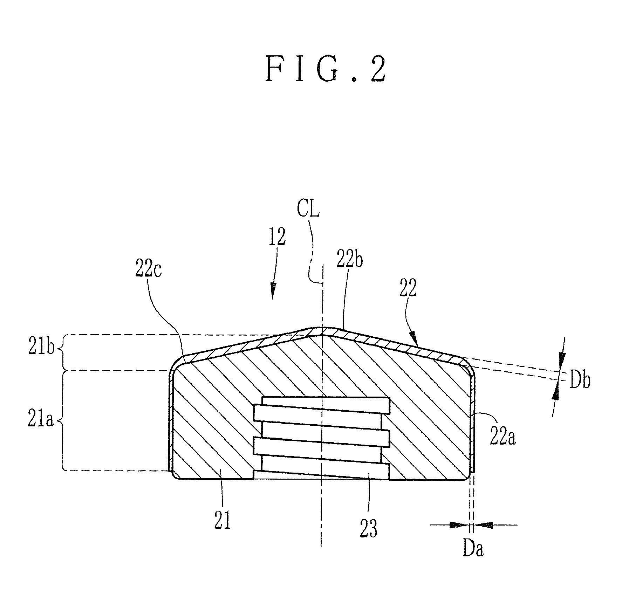

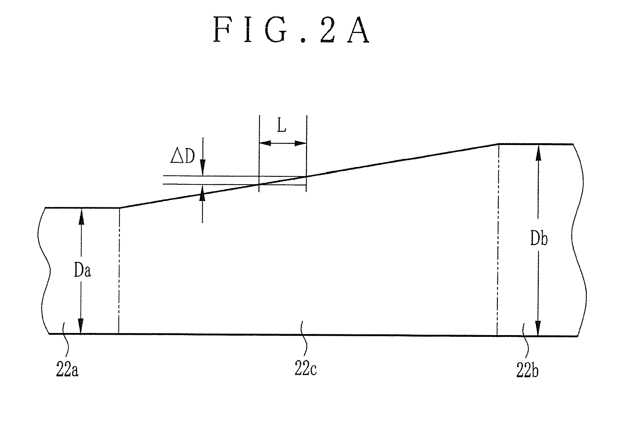

In Fig. 2, the gasket device 12 includes a gasket body 21 (sealing material) and a laminate layer 22 (coating layer). The gasket body 21 has a drum portion 21a or cylindrical portion, and a tapered portion 21b. The drum portion 21a has a constant diameter larger than an inner diameter of the cylinder 11. The tapered portion 21b is formed on a front end of the drum portion 21a with a decreasing diameter. The gasket body 21 is formed from resilient material.

The material for the gasket body 21 is a composition for medical use, for example, a mixture of chlorinated butyl rubber with vulcanizing agents, filler, pigment and antioxidant. Chlorinated butyl rubber (CIIR) is typically preferable for main polymer because of low permeability to oxygen. Note that the main polymer can be butyl rubber (IIR) instead of chlorinated butyl rubber. Specially, isobutylene isoprene copolymer as butyl rubber is preferable for main polymer because of low permeability to oxygen. Also, other compounds can be used for the material of the gasket body 21. For example, silicone rubber is preferable, because of a low content of additives and low risk of dissolution in the liquid drug. Among the various compounds, chlorinated butyl rubber is the most preferable because of the low permeability to oxygen and safety for medical use.

A connecting channel 23 or screw hole is formed in a rear end of the drum portion 21a for attachment of the plunger 14. A female thread is formed inside the connecting channel 23. A male thread 14a is formed on a front end of the plunger 14 as illustrated in Fig. 1, and helically engaged with the female thread in the connecting channel 23. The plunger 14 is coupled to the gasket device 12 by use of the connecting channel 23.

The laminate layer 22 covers surfaces of the drum portion 21a and the tapered portion 21b. The laminate layer 22 includes a slidable sealing portion 22a or sealing membrane, and a liquid receiving portion 22b or stopper portion (plug portion). The sealing portion 22a is positioned around the drum portion 21a, tightly contacts an inner surface 18 of the cylinder 11 and slides thereon. The liquid receiving portion 22b is positioned on the tapered portion 21b, and applies pressure to the liquid drug LM by contacting the same. Note that the laminate layer 22 can be additionally formed on a rear end surface of the drum portion 21a and an inner surface of the connecting channel 23.

The laminate layer 22 is formed from polytetrafluoroethylene or PTFE which has a low frictional resistance and chemically inert property. Other examples of the material for the laminate layer 22 are fluorocarbon resins, such as tetrafluoroethylene/perfluoro(alkyl vinyl ether) copolymer (PFA), ethylene/tetrafluoroethylene copolymer (ETFE) and the like. PTFE is typically preferable among those in view of reducing frictional resistance with the property of a low frictional coefficient.

The gasket body 21 has very high frictional resistance to the inner surface 18 of the cylinder 11 and cannot be slid readily. However, the sealing portion 22a of the laminate layer 22 is kept in contact with the inner surface 18 of the cylinder 11 to facilitate the slide of the gasket device 12. Also, a width of the sealing portion 22a is enlarged as a size in the moving direction A, so that the pre-filled syringe 10 can be constructed with sufficient air-tightness and liquid-tightness. In contrast, the liquid receiving portion 22b prevents the liquid drug LM from contacting the gasket body 21, to prevent a component of the gasket body 21 from dissolving into the liquid drug LM.

Should the thickness Da of the sealing portion 22a be too large, sealing of the gasket body 21 on the inner surface 18 of the cylinder 11 with resiliency will be weak, so that no high sealing tightness (air-tightness and liquid-tightness) can be obtained. Should the thickness Db of the liquid receiving portion 22b be too small, it is likely that pinholes are formed in the liquid receiving portion 22b and that a component in the gasket body 21 may dissolve into the liquid drug LM. Property of inhibiting dissolution of the component will be decreased or lost. Therefore, the sealing portion 22a and the liquid receiving portion 22b are so formed that the thickness Da of the sealing portion 22a is smaller than the thickness Db of the liquid receiving portion 22b. A reason of creation of pinholes is that a film material for the laminate layer 22 may be overstretched in the course of forming. Another reason for the pinholes is that a thickness of the film material for forming at a required thickness is initially small and pinholes may exist prior to the forming.

The laminate layer 22 is so formed as to satisfy Conditions (1), (2) and (3).

$$10 \text{ microns} \leq Da \leq 40 \text{ microns} \ \ldots \text{ Condition (1)}$$

$$20 \text{ microns} \leq Db \leq 50 \text{ microns} \ \ldots \text{ Condition (2)}$$

$$1.25 \leq R1 \leq 2.00 \ ... \ \text{Condition (3)}$$

Condition (1) relates to the thickness Da (first thickness) of the sealing portion 22a. Condition (2) relates to the thickness Db (second thickness) of the liquid receiving portion 22b. Condition (3) relates to R1 = Db/Da as a thickness ratio of the thickness Db of the liquid receiving portion 22b to the thickness Da of the sealing portion 22a.

Should the thickness Da of the sealing portion 22a be smaller than 10 microns, a pinhole or crack occurs in the sealing portion 22a. Thus, it is likely that the laminate layer 22 is torn away upon sliding the gasket device 12. The gasket body 21 may be uncovered upon tearing the laminate layer 22, and to cause high stiction on the inner surface 18 of the cylinder 11, so that it is likely that the plunger 14 cannot be driven for pressure. Also, a small gap may occur between the sealing portion 22a and the inner surface 18 of the cylinder 11 due to a pinhole or crack, to leak a volatile component of rubber in the gasket body 21 from the pinhole or crack, or leak gas or the like in the air. The liquid drug LM may receive the volatile component or air and may be degraded. Should the thickness Da of the sealing portion 22a be larger than 40 microns, resiliency of the gasket body 21 may become ineffective in contact with the inner surface 18 of the cylinder 11, to lower the air-tightness or liquid-tightness.

Should the thickness Db of the liquid receiving portion 22b be smaller than 20 microns, it is likely that pinholes are formed in the liquid receiving portion 22b and that property of inhibiting dissolution will be decreased or lost. Should the thickness Db of the liquid receiving portion 22b be larger than 50 microns, the sealing portion 22a cannot be formed at the thickness Da satisfying Condition (1), to lower the air-tightness.

The laminate layer 22 is formed by press forming of a single PTFE film. A film portion for the sealing portion 22a in the film to have a smaller thickness than the liquid receiving portion 22b is stretched more largely than the liquid receiving portion 22b. Should the thickness ratio R1 be too large, the film portion for the sealing portion 22a in the film may be overstretched to cause whitening, namely, to form fine pinholes or cracks or increase the surface roughness of the PTFE film. Performance in the slip property, sealing tightness, and inhibition of dissolution will be low totally. Should the thickness ratio R1 be too small, there is no effect for balancing the performance. As a thickness difference between the sealing portion 22a and the liquid receiving portion 22b is small, the thickness Da of the sealing portion 22a becomes large according to greatness in the thickness Db of the liquid receiving portion 22b for the purpose of inhibiting dissolution. This causes a drop the sealing tightness. In contrast, a decrease in the thickness Da of the sealing portion 22a for the purpose of increasing the sealing tightness will cause a decrease in the thickness Db of the liquid receiving portion 22b. This makes it difficult to ensure inhibition of dissolution. In conclusion, Condition (3) is obtained in view of those problems to have high performance in the slip property, sealing tightness, and inhibition of dissolution.

It is also preferable to satisfy Condition (4).

$$Sb \leq 1/4 \ ... \ \text{Condition (4)}$$

This relates to a thickness gradient Sb in the thickness of the laminate layer 22 at a transition portion 22c between the sealing portion 22a and the liquid receiving portion 22b. The thickness gradient Sb is defined as a ratio $\Delta D/L$ of a thickness change $\Delta D$ of the laminate layer 22 to a local distance L between two points in the transition portion 22c in a direction along the laminate layer 22. In Fig. 2A, the sealing portion 22a and the liquid receiving portion 22b are virtually extended on a plane.

Condition (4) is for the purpose of preventing an increase in the resistance to the slide, because an abrupt change in the thickness of the transition portion 22c may cause interference with the inner surface 18 of the cylinder 11 at the stepped shape. To be precise, Condition (4) can be satisfied by decreasing the thickness of the transition portion 22c at a thickness change equal to or less than 0.25 micron per one shift (local distance) from the liquid receiving portion 22b toward the sealing portion 22a with 1 micron. The thickness gradient Sb is preferably equal to or less than 1/6, and desirably equal to or less than 1/8.

It is preferable in the laminate layer 22 to set a thickness difference $\Delta Da$ between two symmetric parts of the sealing portion 22a equal to or less than 5 microns, the two symmetric parts being symmetrically opposite to each other with respect to a center line CL of the gasket body 21 as a symmetry axis. This is effective in minimizing deviation in resistance to the slide due to specificity of the gasket device 12 in the pre-filled syringe 10. Should the thickness difference $\Delta Da$ be more than 5 microns, the gasket device 12 may be shifted toward a soft side of the sealing portion 22a with a smaller thickness by resilient deformation upon pushing the plunger 14. Pushing force required for sliding the gasket device 12 may be considerably high to enlarge the deviation in the resistance to the slide. It is conceivable to set the thickness Da of the sealing portion 22a uniform completely for the purpose of reducing the deviation in the resistance to the slide. However, this conception is not practical because producing the gasket device 12 of this structure will increase the manufacturing cost.

However, it has been found that the deviation in the resistance to the slide can be reduced to a low level without a problem in the practical use by setting the thickness difference ΔDa equal to or less than 5 microns. To be precise, in case the thickness difference ΔDa of the sealing portion 22a is more than 5 microns, a maximum range of the deviation is approximately 20 N. In case the thickness difference ΔDa is equal to or less than 5 microns, the maximum range of the deviation is as small as 10 N.

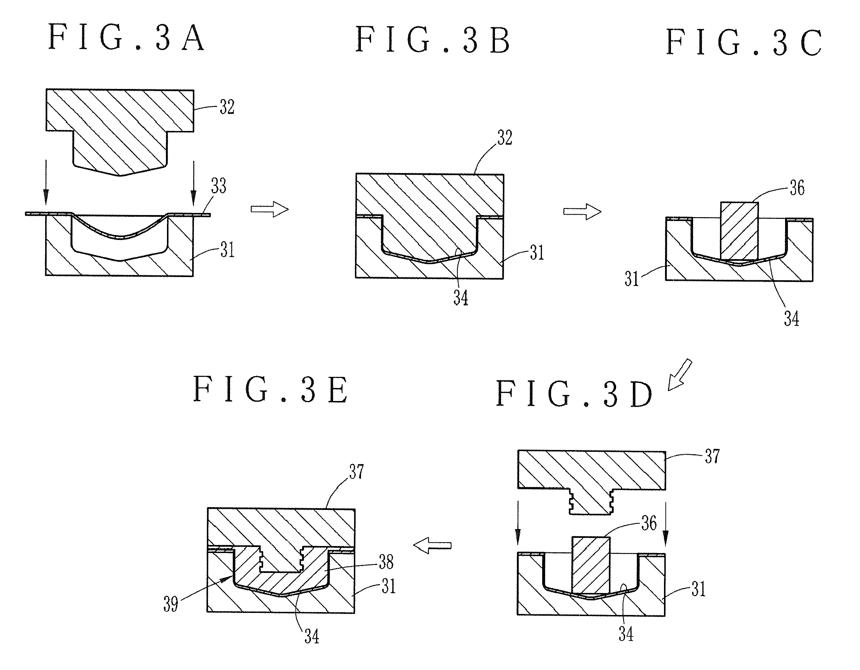

A process of producing the gasket device 12 for the pre-filled syringe 10 is described next. The process of the press forming includes first and second steps. In Fig. 3A, a female mold 31 or cavity mold, and a male mold 32 or core mold are used in the first step, to form a PTFE film 33. The female mold 31 has a cavity defined for a profile of the gasket device 12. The male mold 32 has a core defined for a profile of the gasket body 21. The PTFE film 33 is loosed at a predetermined length, and set between the male and female molds 31 and 32. In Fig. 3B, the male mold 32 is pressed into the female mold 31 to form the PTFE film 33 by applying heat and pressure.

For the common use of the female mold 31 between the first and second steps, the male and female molds 31 and 32 in the first step are heated at 180 degrees Centigrade for forming the PTFE film 33, namely as high as the temperature in the second step for rubber forming of the gasket body 21.

The looseness of the PTFE film 33 in the female mold 31 is adjusted to adjust the thicknesses Da and Db of the sealing portion 22a and the liquid receiving portion 22b as required for the purpose. Note that it is possible to adjust an amount of pull according to a shape of the molds or to adjust tension of the PTFE film 33 at the time of closing the molds, instead of adjusting the looseness of the PTFE film 33.

The PTFE film 33 is stretched by the male mold 32 upon its entry with pressure. A film portion of the PTFE film 33 for the sealing portion 22a is stretched largely, in contrast with another film portion for the liquid receiving portion 22b. Thus, a pressed material 34 is obtained in a shape of the surface of the gasket device 12 with the predetermined thicknesses Da and Db by adjusting the looseness, so that the sealing portion 22a is thinner the liquid receiving portion 22b.

The male mold 32 is raised while the pressed material 34 remains the female mold 31 in Fig. 3C. Then in the second step, a block 36 of chlorinated butyl rubber as a kneaded material is placed in the female mold 31 for the gasket body 21. In Fig. 3D, the female mold 31 is moved to a position directly under a male mold 37 or core mold which has a core for forming the connecting channel 23. Then the male mold 37 is pressed into the female mold 31 and heat is applied as illustrated in Fig. 3E. Then a molded article 39 is molded, inclusive of a molded material 38 prepared for forming the gasket body 21 and the pressed material 34 deposited on a surface of the molded material 38. Consequently, it is possible to reduce the number of molds in the mold set because the female mold 31 functions in the first and second steps. It is unnecessary to move the pressed material 34 to another mold. It is possible to reduce a manufacturing cost and increase the efficiency in the production.

The molded article 39 is cooled, and removed from the female mold 31. Unwanted portions of the molded article 39 are cut away to obtain the gasket device 12.

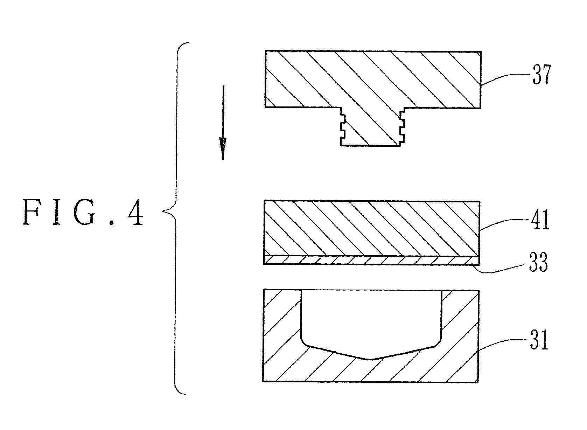

In Fig. 4, another preferred method of producing the gasket device 12 in a single molding step is illustrated. A rubber sheet 41 and the PTFE film 33 are initially placed on the female mold 31. The rubber sheet 41 is a material for the gasket body 21. The male mold 37 is entered into the female mold 31, to which heat is applied for forming the gasket device 12.

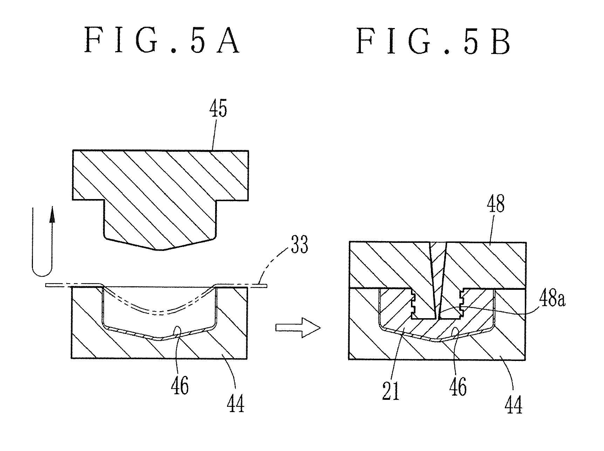

Figs. 5A and 5B illustrate another preferred producing method according to insert molding. In Fig. 5A, at first the PTFE film 33 is placed between a female mold 44 or cavity mold and a male mold 45 or core mold. The male mold 45 is pressed into the female mold 44 to mold the PTFE film 33 into a pressed material 46 in a shape for the surface of the gasket device 12. For the production of the pressed material 46, the steps of Figs. 3A-3E are repeated.

A male mold 48 or core mold has a core for forming the connecting channel 23. The female mold 44 is moved to a position directly under the male mold 48. In Fig. 5B, the male mold 48 is entered in the female mold 44. There is a gate 48a through which the material for the gasket body 21 is injected to form the gasket body 21.

In any of the producing methods in Figs. 3A-5B, it is preferable for the PTFE film 33 to have a thickness equal to or more than 30 microns and equal to or less than 60 microns, and to have a stretch ratio equal to or more than 200 % and equal to or less than 400 % at the yield point of the PTFE film 33 according to the tensile test. Should the PTFE film 33 not satisfy this condition of the stretch ratio, it is difficult to obtain the gasket device with the thickness ratio of the feature of the present invention. It is to be noted that the stretch ratio at the yield point is distinct from the stretch ratio indicated by the thickness ratio of the laminate layer 22.

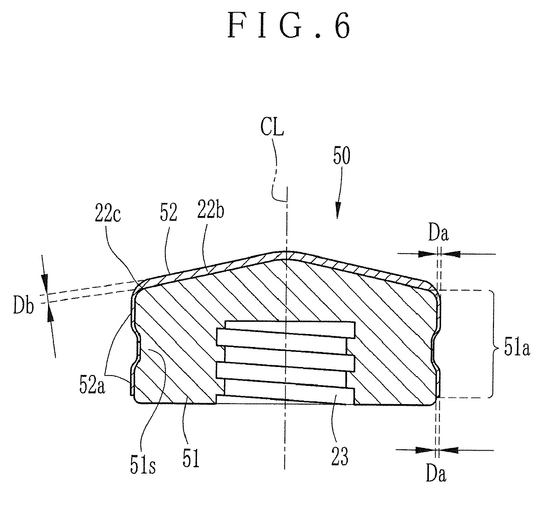

In Fig. 6, another preferred gasket device 50 or plunger stopper (plunger head) includes two slidable sealing portions 52a or sealing membranes. A gasket body 51 (sealing material) includes a drum portion 51a or cylindrical portion, in which a small diameter portion 51s is formed intermediately. A laminate layer 52 (coating layer) has the sealing portions 52a covering first and second ring portions between which the small diameter portion 51s is disposed, so that the sealing portions 52a are slid in the cylinder 11 or barrel. For the gasket device 50, the gasket device 12 is repeated but with a difference in that the sealing portions 52a have the equal thickness Da at two locations.

To produce the gasket device 50, the materials are molded by use of a female mold having a sub-core and a male mold having a sub-cavity.

In the above pre-filled syringe, the plunger is originally separate from the gasket device and fitted thereon to slide for injection. However, a pre-filled syringe of the invention may be a structure in which a plunger is incorporated and a gasket device is originally attached to the plunger.

[Examples 1-7]

The gasket device 12 of Fig. 2 was produced by the producing method of Figs. 3A-3E and evaluated. The gasket device 12 had a diameter of 34.1 mm $\pm$ 0.1 mm, and a total length of 17.1 mm $\pm$ 0.1 mm. The cylindrical portion of the gasket device 12 had a length of 10.0 mm $\pm$ 0 .1 mm. The connecting channel 23 had an inner diameter of 22.4 mm $\pm$ 0.1 mm, and a height of 10.0 mm $\pm$ 0.1 mm. The thickness gradient Sb of any one of Examples 1-7 was 1/5.

In Examples 1-7, the material for the gasket body 21 was chlorinated butyl rubber with vulcanizing agent, filler, pigment and antioxidant. Specifically, CHLOROBUTYL 1066 (chlorination rate 1.26 %, manufactured by Japan Butyl Co., Ltd.) was used as main polymer or rubber, to which the following materials were added: vulcanizing agent of a triazine compound ACTOR TSH (manufactured by Kawaguchi Chemical Industry Co., Ltd.), silicon dioxide as an inorganic filler NIPSIL (manufactured by Tosoh Silica Corporation), and inorganic pigments (titanium oxide and carbon black). A rubber composition containing those was kneaded in an open roll type of kneader, so that the block 36 is produced from the rubber composition obtained by kneading. The block 36 was molded into the gasket body 21. For the material of the laminate layer 22, the PTFE film 33 was used.

In any one of the first and second steps, the female mold 31 and the male molds 32 and 37 were heated at 180 degrees Centigrade, and driven for molding for 10 minutes. The thickness Df of the PTFE film 33 was 60 microns in Examples 1, 2 and 6, 50 microns in Example 3, 40 microns in Example 4, and 30 microns in Examples 5 and 7.

Adjustment of the thicknesses Da and Db of the sealing portion 22a and the liquid receiving portion 22b in Examples 1-7 was carried out by adjusting looseness of the PTFE film 33 in the female mold 31. Specifically, the thicknesses Da and Db and the thickness ratio R1 were set as indicated in Table 1. The thicknesses Da and Db were obtained by cutting the gasket device 12 with a blade of a cutter and measuring a cross section of the gasket device 12 by use of a digital microscope VHX-1000 (manufactured by Keyence Corporation). In the measurement, plural points of each of the sealing portion 22a and the liquid receiving portion 22b were measured, to obtain their average values to indicate the thicknesses Da and Db in Table 1. In relation to the sealing portion 22a, the plural points for the measurement included a first point at a distal end (on a side of the liquid receiving portion 22b), a second point at a proximal end, and a third point defined between the first and second points. In relation to the liquid receiving portion 22b, the plural points for the measurement included a first point at the center of the liquid receiving portion 22b (on the center line CL), and second and third points between which the center line CL is disposed at a location between a peripheral edge and the center. The precision of the thicknesses of the sealing portion 22a and the liquid receiving portion 22b was equal to or less than 1 micron. The thickness ratio R1 was calculated according to the thicknesses Da and Db obtained by the measurement.

Occurrence of crack at the sealing portion 22a and the liquid receiving portion 22b of the laminate layer 22 was examined by a digital microscope VHX-1000 (manufactured by Keyence Corporation). Table 1 indicates results of the crack. The sign "-" denotes that no crack was found. An arithmetic average surface roughness Ra of the surface of the sealing portion 22a was measured and obtained as indicated in Table 1. The arithmetic average surface roughness Ra was measured by a non-contact three-dimensional shape measuring apparatus NH-3N (manufactured by Mitaka Kohki Co., Ltd.) according the standards JIS B 0601:2001.

For the resistance to the slide, the cylinder 11 was filled with liquid electrolyte Otsuka Normal Saline (manufactured by Otsuka Pharmaceutical Co., Ltd.) by way of the liquid drug LM. The plunger 14 was driven for slide with the gasket device 12 in the cylinder 11, where pushing force required for the slide was measured. In the measurement, the pre-filled syringe 10 was set in a tensile tester AUTOGRAPH AGS-X (manufactured by Shimadzu Corporation). For setting this, a tool for holding the pre-filled syringe 10 was prepared in such manner that a pull direction of the tensile tester was set forwards in the moving direction A of the plunger 14. In the measurement, the tip cap 13 was removed, and an injection needle was mounted in the needle holder 11a. Table 1 indicates results of the pushing force.

The air-tightness was measured for the sealing tightness. Note that liquid-tightness was not measured, because testing the air-tightness with gas was more appropriate than testing the liquid-tightness with liquid owing to high sensitivity in detecting leakage. In the measurement, a test slide Fuji Dri-chem Slide NH3-WII (manufactured by Fujifilm Corporation) was entered in the cylinder 11. The gasket device 12 and the tip cap 13 were mounted in the cylinder 11 of the pre-filled syringe 10, which was placed in a test container filled with ammonia gas, and left to stand at 23 degrees Centigrade for 30 minutes. Then the test slide was measured for a colorimetric change at 600 nm by an analyzer Fuji Dri-chem 7000 (manufactured by Fujifilm Corporation).

Evaluation according to the results is indicated in Table 1 with grades A, B and C. Note that grade A was a state in the color of the test slide did not change. Grade B was a state of a change in the color at a level equal to or lower than 30 % according to the examination scale in which a level of 0 % was defined for a state in which the test slide was not colored by reaction with ammonia, and a level of 100 % was defined for a state in which the test slide was completely colored by reaction with ammonia. Grade C was a state of a change in the color at a level higher than 30 %.

The inhibition of dissolution of components of the gasket body 21 to the liquid drug LM was evaluated according to the "Test for Rubber Closure for Aqueous Infusions" in the Japanese Pharmacopoeia, with items including 1. cadmium, 2. metal lead, 3. dissolution test, 4. acute toxicity test, 5. pyrogen test, and 6. hemolysis test. In Table 1, grade A was a state of lower values according to reference levels. Grade B was a state of values tolerable according to the standards. Grade C was a state of values in a range outside a tolerable range according to the standards.

[Comparisons]

The gasket device 12 was produced in Comparisons 1-4 according to the producing method of Figs. 3A-3E similar to Examples 1-7. Various data were measured, calculated or evaluated, including the thicknesses Da and Db, thickness ratio R1, arithmetic average surface roughness Ra, occurrence of crack, pushing force for the gasket device 12 to slide in the cylinder 11, air-tightness, and inhibition of dissolution. Results are indicated in Table 1. The thickness Df of the PTFE film 33 was 60 microns in Comparisons 1-3, and was 30 microns in Comparison 4. In Comparisons 1-4, the size, production and other conditions of the gasket device 12 of Examples 1-7 were repeated but with differences in that the thicknesses Da and Db were adjusted as indicated in Table 1.

Table 1

| | | Examples | | | |
|---|---|---|---|---|---|
| | | 1 | 2 | 3 | 4 |
| Thickness | Df before molding | 60 | 60 | 50 | 40 |
| | Da (sealing portion 22a) | 25 | 40 | 30 | 20 |
| | Db (liquid receiving portion 22b) | 50 | 50 | 40 | 30 |
| Thickness ratio | R1 (Db/Da) | 2.00 | 1.25 | 1.33 | 1.50 |

| | | | | | |
|---|---|---|---|---|---|
| | R2 (Df/Da) | 2.40 | 1.50 | 1.67 | 2.00 |
| | R3 (Df/Db) | 1.20 | 1.20 | 1.25 | 1.33 |
| Crack | Sealing portion 22a | - | - | - | - |
| | Liquid receiving portion 22b | - | - | - | - |
| Arithmetic average surface roughness Ra (sealing portion 22a) | | 0.9 | 0.4 | 0.5 | 0.7 |
| Pushing force (N) or resistance to slide | | 42 | 40 | 39 | 38 |
| Air-tightness | | B | B | B | A |
| Inhibition of dissolution | | A | A | A | A |

| | | Examples | | |
|---|---|---|---|---|
| | | 5 | 6 | 7 |
| Thickness | Df before molding | 30 | 50 | 30 |
| | Da (sealing portion 22a) | 12 | 20 | 15 |
| | Db (liquid receiving | 20 | 40 | 26 |

| | | | | |
|---|---|---|---|---|
| | portion 22b) | | | |
| Thickness ratio | R1 (Db/Da) | 1.67 | 2.00 | 1.73 |
| | R2 (Df/Da) | 2.50 | 2.50 | 2.00 |
| | R3 (Df/Db) | 1.50 | 1.25 | 1.15 |
| Crack | Sealing portion 22a | - | - | - |
| | Liquid receiving portion 22b | - | - | - |
| Arithmetic average surface roughness Ra (sealing portion 22a) | | 1.0 | 0.8 | 0.3 |
| Pushing force (N) or resistance to slide | | 35 | 40 | 37 |
| Air-tightness | | A | B | A |
| Inhibition of dissolution | | A | A | A |

| | | Comparisons | | | |
|---|---|---|---|---|---|
| | | 1 | 2 | 3 | 4 |
| Thickness | Df before molding | 60 | 60 | 60 | 30 |
| | Da (sealing | 20 | 50 | 20 | 10 |

| | | | | | |
|---|---|---|---|---|---|
| | portion 22a) | | | | |
| | Db (liquid receiving portion 22b) | 50 | 55 | 45 | 15 |
| Thickness ratio | R1 (Db/Da) | 2.50 | 1.10 | 2.25 | 1.50 |
| | R2 (Df/Da) | 3.00 | 1.20 | 3.00 | 3.00 |
| | R3 (Df/Db) | 1.20 | 1.09 | 1.33 | 2.00 |
| Crack | Sealing portion 22a | Occurred | - | Occurred | Occurred |
| | Liquid receiving portion 22b | - | - | - | - |
| Arithmetic average surface roughness Ra (sealing portion 22a) | | 1.2 | 0.3 | 1.1 | 0.7 |
| Pushing force (N) or resistance to slide | | 62 | 40 | 65 | 40 |
| Air-tightness | | C | C | C | A |
| Inhibition of dissolution | | C | A | C | C |

According to the results indicated in Table 1, the thickness of the sealing portion 22a was increased in case the thickness ratio R1 was smaller than the lower limit 1.25 of Condition (2) and in case the thickness Db of the liquid receiving portion 22b was increased for the purpose of ensuring property of inhibiting dissolution. In conclusion, it was difficult to ensure the sealing tightness. It is supposed that this was because the sealing portion 22a was not fitted on the inner surface 18 of the cylinder 11 due to insufficient flexibility, to create gaps between the gasket device 12 and the cylinder 11.

In case the thickness ratio R1 was more than 2.00 as an upper limit of Condition (2), a great number of fine cracks were created by overstretch of the sealing portion 22a, to cause whitening of the laminate layer 22. All of the slip property, sealing tightness, and inhibition of dissolution of the gasket device 12 were found poor.

Note that the thickness ratio R2 (= Df/Da) is a ratio of stretch of the sealing portion 22a from the PTFE film 33 in an original form. The thickness ratio R3 (= Df/Db) is a ratio of stretch of the liquid receiving portion 22b from the PTFE film 33 in an original form. In case the initial thickness Df is equal to or more than 30 microns and equal to or less than 60 microns, no effect for balancing the performance is obtained assuming that the thickness ratio R2 is less than 1.5. Thus, the thickness ratio R2 should be equal to or more than 1.50. However, it is further necessary that the thickness ratio R2 should be less than 3.00 for the purpose of preventing occurrence of crack due to overstretch. The condition of R1 < R2 is satisfied in consideration of the liquid receiving portion 22b is considerably stretched from the form of the PTFE film 33. As the liquid receiving portion 22b does not normally require stretch and should not be overstretched, it is preferable that the thickness ratio R3 should be equal to or less than 1.50. In Table 1, the values for the thickness ratios R2 and R3 were calculated according to the initial thickness Df and the measured thicknesses Da and Db.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

**Claims**

1. A gasket device for a pre-filled syringe including a cylinder (11) for containing liquid drug (LM) in sealing tightness, said liquid drug being pressurized by moving a plunger (14) in said cylinder, said gasket device comprising:

   a gasket body (21, 51), having resiliency, and movable toward said liquid drug with a front end (14a) of said plunger;
   a laminate layer (22, 52), formed from fluorocarbon resin, and disposed to cover a surface of said gasket body; wherein said laminate layer includes:

   a sealing portion (22a, 52a), having a first thickness (Da), for sliding on an inner surface (18) of said cylinder in sealing tightness;
   a liquid receiving portion (22b), having a second thickness (Db), for contacting said liquid drug in said cylinder; wherein said first thickness is equal to or more than 10 microns and equal to or less than 40 microns, said second thickness is equal to or more than 20 microns and equal to or less than 50 microns, and a ratio (R1) of said second thickness to said first thickness is equal to or more than 1.25 and equal to or less than 2.00.

2. A gasket device as defined in claim 1, wherein said laminate layer includes a transition portion formed between said sealing portion and said liquid receiving portion; said transition portion is so formed as to satisfy a condition:

$$\Delta D/L \le 1/4$$

   where L is a local distance between two points arranged in said transition portion close to one another in a direction of transition, and $\Delta D$ is a thickness change in said transition portion between said two points.

3. A gasket device as defined in claim 1 or 2, wherein said laminate layer is in a film form, and shaped in compliance with a profile line of said gasket body by press forming of said fluorocarbon resin.

4. A gasket device as defined in any one of claims 1 to 3, wherein said first thickness is smaller than said second thickness, and a thickness difference between parts of said sealing portion symmetrically opposite to each other with respect to a center line of said gasket body is equal to or less than 5 microns.

5. A gasket device as defined in any one of claims 1 to 4, wherein said gasket body is formed from rubber.

6. A gasket device as defined in any one of claims 1 to 5, wherein said gasket body includes:

first and second ring portions arranged in a moving direction of said plunger;
a small diameter portion disposed between said first and second ring portions in a layer arrangement, and having a smaller diameter than said first and second ring portions;
said sealing portion is formed on a peripheral surface of each one of said first and second ring portions.

7. A gasket device as defined in any one of claims 1 to 6, wherein said laminate layer is formed by press forming, and thereafter said gasket body is formed together with said laminate layer.

8. A gasket device as defined in any one of claims 1 to 6, wherein said laminate layer is formed simultaneously with forming of said gasket body.

9. A gasket device as defined in any one of claims 1 to 6, wherein said gasket body is formed by insert molding on said laminate layer.

# F I G . 1

# F I G . 2

# FIG.2A

FIG.3A

FIG.3B

FIG.3C

FIG.3D

FIG.3E

F I G . 4

37

41

33

31

# FIG.5A

# FIG.5B

# F I G . 6

Europäisches
Patentamt

European
Patent Office

Office européen
des brevets

**EUROPEAN SEARCH REPORT**

Application Number

EP 14 16 5978

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | JP 2008 154644 A (NITTO DENKO CORP)<br>10 July 2008 (2008-07-10)<br>* claims 1-4; figure 2 * | 1<br>2-9 | INV.<br>A61M5/315 |
| X<br>Y | US 2012/123345 A1 (FELTS JOHN T [US] ET AL) 17 May 2012 (2012-05-17)<br>* paragraph [0380]; figure 6 * | 1<br>2-9 | |
| X<br>Y | EP 0 375 778 A1 (TERUMO CORP [JP])<br>4 July 1990 (1990-07-04)<br>* figures * | 1<br>2-9 | |
| Y | EP 1 317 937 A1 (TERUMO CORP [JP]; KOKOKU INTECH CO LTD [JP])<br>11 June 2003 (2003-06-11)<br>* page 6, paragraph 54 * | 2-9 | |
| Y | WO 2011/080543 A1 (BECTON DICKINSON FRANCE [FR]; RODRIGUEZ NESTOR [US]; MANGIAGALLI PAOLO) 7 July 2011 (2011-07-07)<br>* page 19, line 24 - line 27; figure 12 * | 3-5 | TECHNICAL FIELDS SEARCHED (IPC)<br><br>A61M |

The present search report has been drawn up for all claims

| Place of search | Date of completion of the search | Examiner |
|---|---|---|
| Munich | 18 August 2014 | Ehrsam, Fernand |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 14 16 5978

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2014

| Patent document cited in search report | | Publication date | Patent family member(s) | | Publication date |
|---|---|---|---|---|---|
| JP 2008154644 | A | 10-07-2008 | JP | 4860455 B2 | 25-01-2012 |
| | | | JP | 2008154644 A | 10-07-2008 |
| US 2012123345 | A1 | 17-05-2012 | NONE | | |
| EP 0375778 | A1 | 04-07-1990 | AU | 623598 B2 | 21-05-1992 |
| | | | CA | 1324545 C | 23-11-1993 |
| | | | DE | 3883985 D1 | 14-10-1993 |
| | | | DE | 3883985 T2 | 27-01-1994 |
| | | | EP | 0375778 A1 | 04-07-1990 |
| | | | ES | 2007937 A6 | 01-07-1989 |
| | | | WO | 8810130 A1 | 29-12-1988 |
| EP 1317937 | A1 | 11-06-2003 | AU | 8625401 A | 26-03-2002 |
| | | | EP | 1317937 A1 | 11-06-2003 |
| | | | JP | 2002086481 A | 26-03-2002 |
| | | | JP | 2002089717 A | 27-03-2002 |
| | | | US | 2004084852 A1 | 06-05-2004 |
| | | | US | 2005212222 A1 | 29-09-2005 |
| | | | WO | 0222192 A1 | 21-03-2002 |
| WO 2011080543 | A1 | 07-07-2011 | EP | 2519291 A1 | 07-11-2012 |
| | | | US | 2013211344 A1 | 15-08-2013 |
| | | | WO | 2011080543 A1 | 07-07-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010142573 A

- JP 2004248985 A